# EUROPEAN PATENT APPLICATION

(11) **EP 4 821 593 A1**
(43) Date of publication of application: **14.10.2026**
(21) Application number: 25767731.0
(22) Date of filing: 27.02.2025
(51) Int. Cl.: B01J 20/06, A61L 9/01, B01D 21/00, B01D 21/01, B01D 53/02, B01D 53/52, B01J 20/30, C01G 49/02, C02F 1/52, C02F 1/64

(54) **IRON OXYHYDROXIDE-BASED HYDROGEN SULFIDE REMOVING AGENT, METHOD FOR PRODUCING IRON OXYHYDROXIDE-BASED HYDROGEN SULFIDE REMOVING AGENT, AND DESULFURIZATION METHOD**

(30) Priority: 06.03.2024 JP 2024034293
(71) Applicant: JFE Steel Corporation, Tokyo 100-0011 (JP)
(72) Inventor: MATSUYAMA Naoki, Tokyo 100-0011 (JP); YOSHIKAWA Kohei, Tokyo 100-0011 (JP); KOIBUCHI Shun, Tokyo 100-0011 (JP); KAWANO Takashi, Tokyo 100-0011 (JP); SHIGAKI Nobuyuki, Tokyo 100-0011 (JP)
(74) Representative: Grünecker Patent- und Rechtsanwälte PartG mbB
(86) International application number: PCT/JP2025/007026
(87) International publication number: WO 2025/187540

(57) **Abstract**

Provided is a hydrogen sulfide removal agent that can be used stably while maintaining hydrogen sulfide removal performance equivalent to that of a hydrogen sulfide removal agent composed of amorphous iron oxyhydroxide. It is an iron oxyhydroxide-based hydrogen sulfide removal agent containing amorphous iron oxyhydroxide and a crystalline component, where the content of the amorphous iron oxyhydroxide is equal to or greater than the content of the crystalline component.

## Description

### TECHNICAL FIELD

This disclosure relates to an iron oxyhydroxide-based hydrogen sulfide removal agent and a method of producing the same. This disclosure also relates to a desulfurization method using the iron oxyhydroxide-based hydrogen sulfide removal agent.

### BACKGROUND

Gases generated in various facilities such as iron and steel works, petrochemical plants, and sewage treatment facilities contain hydrogen sulfide (H₂S), which causes malodor and corrosion of piping. Accordingly, there is a demand for technologies for removing hydrogen sulfide contained in such gases.

Such gases often also contain a large amount of CO₂, and from the viewpoint of reducing CO₂ emissions, technologies have been proposed in which CO₂ in the gas is converted into methanol or the like by catalytic reactions and thereby recycled as a resource. However, when hydrogen sulfide is contained in the gas, the catalyst deteriorates. Therefore, to prevent catalyst deterioration as well, there is a demand for technologies for removing hydrogen sulfide contained in the gas.

Accordingly, methods have been proposed in which hydrogen sulfide in a gas is adsorbed and removed using a dry hydrogen sulfide removal agent composed of iron oxyhydroxide (FeOOH), such as α-FeOOH (goethite) or β-FeOOH (akaganeite) (for example, PTL 1 and PTL 2). Because iron oxyhydroxide has a large number of basic OH groups on its surface, it adsorbs acidic H₂S through acid-base interactions. As a result, iron oxyhydroxide exhibits superior performance as a hydrogen sulfide removal agent compared with Fe₂O₃ and the like.

However, the removal rate of hydrogen sulfide is still insufficient, and further improvement in performance has been required for use in applications such as iron and steel works, petrochemical plants, and sewage treatment facilities.

Accordingly, in place of crystalline iron oxyhydroxides such as goethite and akaganeite, a hydrogen sulfide removal agent containing amorphous iron oxyhydroxide has been proposed (PTL 3). According to PTL 3, because amorphous iron oxyhydroxide is not crystallized, it has a large surface area that contributes to reactions with sulfur compounds. As a result, compared with akaganeite and the like having a crystalline structure, the reaction rate with sulfur compounds is high, and low-concentration sulfur compounds can be completely removed.

### CITATION LIST

### Patent Literature

PTL 1: JP H05-329362 A
PTL 2: JP 2014-213281 A
PTL 3: JP 2017-177052 A

### SUMMARY

### (Technical Problem)

As proposed in PTL 3, excellent hydrogen sulfide removal performance can be obtained by using amorphous iron oxyhydroxide.

However, such a hydrogen sulfide removal agent composed of amorphous iron oxyhydroxide has a problem in that it has low strength and cannot be used stably. Specifically, as described in PTL 3, the hydrogen sulfide removal agent composed of amorphous iron oxyhydroxide is produced by reacting an aqueous iron chloride solution with an alkaline aqueous solution (neutralizing agent) and hydrogen peroxide (oxidizing agent). At that time, since crystal growth cannot be allowed in order to obtain an amorphous structure, the resulting amorphous iron oxyhydroxide inevitably forms an aggregate of extremely fine particles. A hydrogen sulfide removal agent composed of such fine particles has low strength, and when packed into a desulfurization tower, it cannot withstand its own weight and is liable to collapse and pulverize.

It could thus be helpful to provide a hydrogen sulfide removal agent that can be used stably while maintaining hydrogen sulfide removal performance equivalent to that of a hydrogen sulfide removal agent composed of amorphous iron oxyhydroxide.

### (Solution to Problem)

The present disclosure has been made to solve the above problems, and its primary features are as follows.
1. An iron oxyhydroxide-based hydrogen sulfide removal agent comprising amorphous iron oxyhydroxide and a crystalline component, wherein
   a content of the amorphous iron oxyhydroxide is equal to or greater than a content of the crystalline component.
2. The iron oxyhydroxide-based hydrogen sulfide removal agent according to 1., wherein the content of the amorphous iron oxyhydroxide is 50 wt% to 80 wt%.
3. The iron oxyhydroxide-based hydrogen sulfide removal agent according to 1. or 2., comprising crystalline iron oxide as at least a part of the crystalline component.
4. A method of producing an iron oxyhydroxide-based hydrogen sulfide removal agent, which is a method of producing the iron oxyhydroxide-based hydrogen sulfide removal agent according to any one of 1. to 3., comprising:
   a neutralization process of neutralizing an aqueous solution containing iron ions to precipitate an iron component;
   a flocculation and sedimentation process of flocculating and subjecting to sedimentation the iron component obtained in the neutralization process, and removing a supernatant to obtain an iron hydroxide-containing slurry;
   a solid-liquid separation process of subjecting the iron hydroxide-containing slurry to solid-liquid separation to obtain an iron oxyhydroxide-containing cake; and
   a drying process of drying the iron oxyhydroxide-containing cake to obtain iron oxyhydroxide.
5. The method of producing an iron oxyhydroxide-based hydrogen sulfide removal agent according to 4., wherein the aqueous solution containing iron ions is a waste liquid containing iron ions.
6. The method of producing an iron oxyhydroxide-based hydrogen sulfide removal agent according to 4. or 5., wherein a calcium compound is used as a neutralizing agent in the neutralization process.
7. The method of producing an iron oxyhydroxide-based hydrogen sulfide removal agent according to any one of 4. to 6., wherein the pH is adjusted to 6 to 8 in the neutralization process.
8. The method of producing an iron oxyhydroxide-based hydrogen sulfide removal agent according to any one of 4. to 7., wherein an anionic polymer is used as a flocculant in the flocculation and sedimentation process.
9. A desulfurization method of removing hydrogen sulfide contained in a gas using the iron oxyhydroxide-based hydrogen sulfide removal agent according to any one of 1. to 3.

### (Advantageous Effect)

The hydrogen sulfide removal agent of the present disclosure contains amorphous iron oxyhydroxide in an amount equal to or greater than that of a crystalline component, and as a result, exhibits hydrogen sulfide removal performance equivalent to that of conventional hydrogen sulfide removal agents composed of amorphous iron oxyhydroxide. In addition, since the hydrogen sulfide removal agent of the present disclosure contains a crystalline component in addition to amorphous iron oxyhydroxide, it has a smaller specific surface area than conventional hydrogen sulfide removal agents composed of amorphous iron oxyhydroxide, that is, a larger particle size. Accordingly, it has high strength and can be used stably. As described above, the hydrogen sulfide removal agent of the present disclosure has excellent stability while maintaining hydrogen sulfide removal performance equivalent to that of conventional hydrogen sulfide removal agents composed of amorphous iron oxyhydroxide, and thus can be very suitably used for the removal of hydrogen sulfide.

### BRIEF DESCRIPTION OF THE DRAWINGS

In the accompanying drawings:
FIG. 1 is a graph illustrating the correlation between the proportion of amorphous iron oxyhydroxide contained in a hydrogen sulfide removal agent and the sulfur adsorption amount per unit weight;
FIG. 2 is a graph illustrating an example of the cumulative pore volume distribution of a hydrogen sulfide removal agent; and
FIG. 3 is a graph illustrating the correlation between the proportion of amorphous iron oxyhydroxide contained in a hydrogen sulfide removal agent and the sulfur adsorption amount per unit surface area.

### DETAILED DESCRIPTION

The following describes embodiments of the present disclosure in detail.

### [Hydrogen Sulfide Removal Agent]

An iron oxyhydroxide-based hydrogen sulfide removal agent according to one embodiment of the present disclosure contains amorphous iron oxyhydroxide and a crystalline component, where the content of the amorphous iron oxyhydroxide is equal to or greater than the content of the crystalline component. The reason for this limitation will be described below.

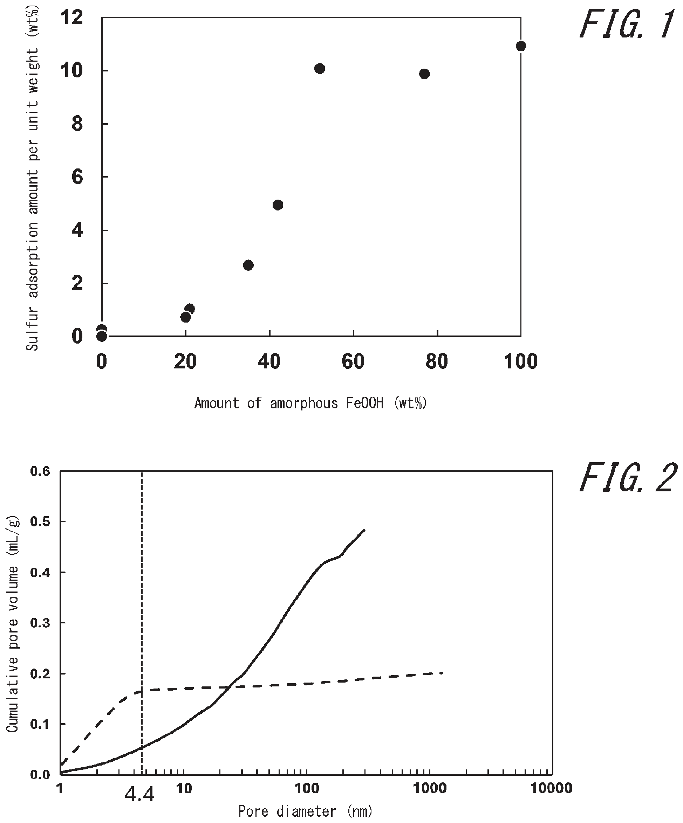

As described above, the hydrogen sulfide removal agent proposed in PTL 3 is composed of amorphous iron oxyhydroxide and does not contain crystalline iron oxyhydroxide. This is evident from the fact that, as described in paragraphs [0023] and [0028] and FIG. 1 of PTL 3, only diffraction peaks of sodium chloride as an impurity are observed in X-ray diffraction.

When such amorphous iron oxyhydroxide is produced, crystal growth cannot be allowed. Therefore, the particles constituting the amorphous iron oxyhydroxide are inevitably fine particles. As a result, there has been a problem in that the strength is low and stable use is not possible.

In contrast, a crystalline component is obtained by allowing crystals to grow, and therefore has a larger particle size than amorphous iron oxyhydroxide. Accordingly, by containing the crystalline component in addition to amorphous iron oxyhydroxide, the hydrogen sulfide removal agent exhibits improved strength and can be used stably. The difference in particle size of the particles contained in the hydrogen sulfide removal agent can be evaluated based on the specific surface area, as described later.

However, in PTL 3, the hydrogen sulfide removal performance is improved by utilizing the fact that fine amorphous iron oxyhydroxide has a large specific surface area. Based on this knowledge of the prior art, it would be presumed that addition of a crystalline component having larger particle size would decrease the specific surface area and, as a result, also decrease the hydrogen sulfide removal performance.

However, as a result of studies, we have found that even when a crystalline component is present in the hydrogen sulfide removal agent, if the content of amorphous iron oxyhydroxide is equal to or greater than the content of the crystalline component, it is possible to maintain hydrogen sulfide removal performance substantially equivalent to that of a hydrogen sulfide removal agent composed of amorphous iron oxyhydroxide.

For example, FIG. 1 is a graph illustrating the correlation between the proportion of amorphous iron oxyhydroxide (FeOOH) contained in the hydrogen sulfide removal agent and the sulfur adsorption amount per unit weight. The amount of amorphous iron oxyhydroxide and the sulfur adsorption amount per unit weight were measured with the methods described in the Examples section below. The remainder other than amorphous iron oxyhydroxide is substantially a crystalline component.

As can be seen from this graph, when the content of the amorphous iron oxyhydroxide is equal to or greater than the content of the remaining crystalline component, it is possible to maintain performance substantially equivalent to that when the content of the amorphous iron oxyhydroxide is 100 %. Therefore, in the present disclosure, the content of the amorphous iron oxyhydroxide is set to be equal to or greater than the content of the crystalline component. The content of the amorphous iron oxyhydroxide is preferably 50 wt% to 80 wt%. The content of the amorphous iron oxyhydroxide can be measured by a combination of the Karl Fischer method and X-ray diffraction measurement. Specifically, the content of the amorphous iron oxyhydroxide can be determined by subtracting the content of crystalline iron oxyhydroxide obtained by X-ray diffraction measurement from the content of iron oxyhydroxide obtained using the Karl Fischer method. More specifically, it can be determined with the method described in the Examples section.

The reason why excellent desulfurization performance is maintained even when a certain amount of crystalline component is contained in the hydrogen sulfide removal agent is considered as follows.

First, the reaction that occurs when hydrogen sulfide is brought into contact with iron oxyhydroxide is represented by the following equation (1).

2FeOOH + 3H₂S → Fe₂S₃ + 4H₂O (1)

Since the above reaction occurs on the surface of the hydrogen sulfide removal agent, the surface area of the hydrogen sulfide removal agent affects the desulfurization performance.

However, since Fe₂S₃ produced by the above reaction is unstable, it is converted into FeS and elemental sulfur by the reaction indicated in the following equation (2). The generated FeS and elemental sulfur accumulate on the surface of the hydrogen sulfide removal agent.

Fe₂S₃ → 2FeS + S (2)

FIG. 2 is a graph illustrating an example of the cumulative pore volume distribution of the hydrogen sulfide removal agent. The dashed line represents measured values for a hydrogen sulfide removal agent composed of amorphous iron oxyhydroxide (Comparative Example No. 4 described later in the Examples section), and the solid line represents measured values for a hydrogen sulfide removal agent containing 48 wt% of a crystalline component (Example No. 1 described later in the Examples section). The specific surface area was measured with the method described in the Examples section.

As can be seen from this graph, the pores present in the hydrogen sulfide removal agent composed of amorphous iron oxyhydroxide are mainly fine pores having a diameter of 4.4 nm or less, and pores having a diameter exceeding 4.4 nm are scarcely present. This is presumably because the particles of amorphous iron oxyhydroxide themselves are extremely fine. The value of 4.4 nm corresponds to approximately ten times the diameter of a hydrogen sulfide molecule.

As described above, because the pores present in the hydrogen sulfide removal agent composed of amorphous iron oxyhydroxide are extremely fine, they are considered to be easily blocked by sulfur and the like generated in the reaction of the equation (2), and thus become unable to contribute to the removal of hydrogen sulfide. As a result, the hydrogen sulfide removal agent composed of amorphous iron oxyhydroxide has a low sulfur adsorption amount per unit surface area.

On the other hand, in a hydrogen sulfide removal agent containing a crystalline component, the proportion of pores having larger diameters is significantly increased compared with the hydrogen sulfide removal agent composed of amorphous iron oxyhydroxide. Therefore, in the hydrogen sulfide removal agent containing a crystalline component, blockage of pores by generated sulfur and the like is less likely to occur, and as a result, high desulfurization performance can be maintained.

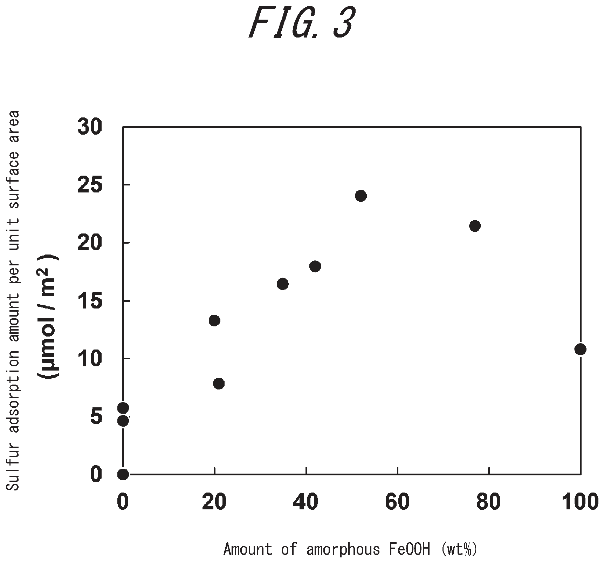

For example, FIG. 3 is a graph illustrating the correlation between the proportion of amorphous iron oxyhydroxide contained in the hydrogen sulfide removal agent and the sulfur adsorption amount per unit surface area under the same conditions as in FIG. 1. The amount of amorphous iron oxyhydroxide and the sulfur adsorption amount per unit surface area were measured with the methods described in the Examples section below.

As can be seen from this graph, the sulfur adsorption amount per unit surface area is actually increased when a certain amount of crystalline component is contained, compared with the case where the proportion of amorphous iron oxyhydroxide is 100 wt%.

However, when the proportion of amorphous iron oxyhydroxide is less than 50 wt%, the sulfur adsorption amount per unit surface area decreases. This is because the proportion of amorphous iron oxyhydroxide capable of adsorbing hydrogen sulfide on the surface of the hydrogen sulfide removal agent decreases. Therefore, from this viewpoint, it is preferable that the content of the amorphous iron oxyhydroxide be 50 wt% or more.

### (Crystalline Component)

The crystalline component is not particularly limited, and may be any substance having a crystalline structure. The crystalline component may typically be at least one selected from the group consisting of crystalline oxides, crystalline hydroxides, and crystalline metal salts.

The crystalline oxide may be either a crystalline metal oxide or a crystalline nonmetal oxide. Examples of the crystalline oxide include at least one oxide selected from the group consisting of Fe, Co, Ni, Ti, V, Cr, Zr, Nb, Mo, Hf, Ta, W, Mg, Al, Zn, Ca, Mn, Cu, and Si.

Examples of the crystalline hydroxide include at least one hydroxide selected from the group consisting of Fe, Al, Mg, Zn, Ni, Mn, and Cr.

Examples of the crystalline metal salt include metal carbonates and metal sulfates. For example, as described later, when a calcium compound such as calcium hydroxide (Ca(OH)₂) or calcium carbonate (CaCO₃) is used as a neutralizing agent during the production process, the finally obtained hydrogen sulfide removal agent contains crystalline calcium carbonate. Similarly, when a Na compound such as NaOH is used as a neutralizing agent, the finally obtained hydrogen sulfide removal agent contains crystalline sodium carbonate (Na₂CO₃). Further, when sulfate ions are present in the aqueous solution, crystalline sulfates such as iron sulfate and calcium sulfate may be contained.

In the present disclosure, it is sufficient that the content of amorphous iron oxyhydroxide be equal to or greater than the content of the crystalline component. In other words, the content of the crystalline component only needs to be less than the content of amorphous iron oxyhydroxide. The preferred content of the crystalline component is 5 wt% to 50 wt%. The content of the crystalline component can be measured by X-ray diffraction measurement. More specifically, it can be measured with the method described in the Examples section.

### (Specific Surface Area)

As described above, since the hydrogen sulfide removal agent of the present disclosure contains a crystalline component, it has a larger particle size than conventional hydrogen sulfide removal agents composed of amorphous iron oxyhydroxide. Such differences in particle size can be evaluated based on the specific surface area. Specifically, it can be said that the smaller the specific surface area, the larger the particle size.

The specific surface area of the iron oxyhydroxide-based hydrogen sulfide removal agent according to the present disclosure is not particularly limited, but it is preferably 100 m²/g or more, more preferably 110 m²/g or more, and still more preferably 120 m²/g or more. On the other hand, the upper limit of the specific surface area is also not particularly limited, but it is typically preferably 200 m²/g or less, more preferably 180 m²/g or less, and still more preferably 160 m²/g or less.

Here, the specific surface area refers to the BET specific surface area measured using nitrogen gas. More specifically, it can be measured with the method described in the Examples section.

### [Production Method]

Next, a method of producing the iron oxyhydroxide-based hydrogen sulfide removal agent according to one embodiment of the present disclosure will be described. The production method of the present embodiment includes the following processes (1) to (4). Each process will be described below.
(1) Neutralization process
(2) Flocculation and sedimentation process
(3) Solid-liquid separation process
(4) Drying process

### (1) Neutralization Process

First, an aqueous solution containing iron ions is neutralized to precipitate an iron component (neutralization process). The iron ions are not particularly limited, and may be either divalent iron ions or trivalent iron ions.

For example, when trivalent iron ions are contained in the aqueous solution, the iron ions precipitate as iron hydroxide as represented by the following equation (3):

Fe³⁺ + 3OH⁻ → Fe(OH)₃↓ (3)

In the equation (3) above, the stoichiometric ratio (molar ratio) of Fe to OH is 1:3. However, in an actual reaction, the ratio is not necessarily 1:3.

Further, when divalent iron ions are contained in the aqueous solution, it is preferable to oxidize the divalent iron ions in advance to convert them into trivalent iron ions. The method of oxidizing the divalent iron ions is not particularly limited, but a method in which a gas containing oxygen (preferably air) is bubbled through the aqueous solution is particularly preferably used.

Any aqueous solution containing the iron ions may be used. However, from the viewpoint of effectively utilizing resources, it is preferable to use waste liquid containing iron ions. The type of the waste liquid is not particularly limited. For example, waste liquid generated during an ironmaking process in steelworks can be suitably used because it contains a sufficient amount of iron ions.

The waste liquid generated during the ironmaking process in steelworks is not particularly limited, and any waste liquid containing iron ions may be used. Examples of the waste liquid include pickling waste liquid generated in a pickling process for a steel sheet surface, and rinse waste liquid generated in a rinsing process in which the steel sheet surface is washed with water after the pickling process. In steelworks, waste liquids generated in a plurality of processes are generally recovered in a mixed state and collectively treated. Therefore, in the present disclosure, a mixed waste liquid obtained by mixing waste liquids generated in a plurality of processes in steelworks may be used.

Further, the waste liquid generated during the ironmaking process may contain solid matter such as dust. As the aqueous solution containing iron ions in the present disclosure, waste liquid containing such solid matter may be used. When the aqueous solution containing iron ions contains solid matter, at least a part of the solid matter is mixed as an impurity into the iron hydroxide-containing slurry recovered in the flocculation and sedimentation process. However, so long as the content of amorphous iron oxyhydroxide in the finally obtained iron oxyhydroxide-based hydrogen sulfide removal agent satisfies the above-described conditions, there is no problem in the function as a hydrogen sulfide removal agent.

The pH in the neutralization process is not particularly limited. However, when the pH is excessively low, Fe ions may become stable in the aqueous solution, and iron hydroxide may not sufficiently precipitate. Therefore, the pH is preferably 6 or more. On the other hand, when the pH is excessively high, the generated iron hydroxide may crystallize and change into goethite (α-FeOOH), which has low desulfurization performance. Therefore, the pH is preferably 8 or less.

To perform neutralization, a neutralizing agent may be added to the aqueous solution containing iron ions. Any neutralizing agent may be used without limitation. However, when a strongly basic substance such as sodium hydroxide (NaOH) is used as the neutralizing agent, crystallization of iron hydroxide may proceed. This is presumably because, when NaOH is added to the aqueous solution, the NaOH is not uniformly mixed, and local regions in which the pH exceeds 8 are generated. On the other hand, when a calcium compound such as CaCO₃ or Ca(OH)₂ is used as the neutralizing agent, the pH can be increased gradually. As a result, it can prevent the pH from becoming excessively high in localized areas. Furthermore, calcium compounds are less expensive than NaOH, and therefore can reduce the production cost of the hydrogen sulfide removal agent. For the above reasons, it is preferable to use a calcium compound as the neutralizing agent, and it is more preferable to use either or both of CaCO₃ and Ca(OH)₂.

Among calcium compounds, Ca(OH)₂ is excellent in neutralizing action. On the other hand, although CaCO₃ has weak neutralizing action, it has a function of assisting flocculation of iron hydroxide by linking generated iron hydroxide particles together. Therefore, it is more preferable to use Ca(OH)₂ and CaCO₃ in combination as the calcium compound.

Further, neutralization may be performed in multiple stages using two or more neutralizing agents. Among these methods, it is preferable to perform neutralization in two stages in which CaCO₃ is first added, followed by addition of Ca(OH)₂. According to this method, the amount of Ca(OH)₂ used can be reduced. In addition, by adding CaCO₃ first, precipitation is more likely to occur with CaCO₃ serving as nuclei.

### (2) Flocculation and Sedimentation Process

Next, the iron component obtained in the neutralization process is flocculated and sedimented, and the supernatant is removed to obtain an iron hydroxide-containing slurry (flocculation and sedimentation process). The method of flocculation and sedimentation is not particularly limited, but typically, a flocculant may be added to the aqueous solution after neutralization.

Any flocculant may be used without limitation, but an anionic polymer is preferably used as the flocculant. Specifically, the iron hydroxide precipitated in the neutralization process exists as positively charged colloidal particles and is dispersed in the aqueous solution due to repulsive forces between the positive charges. Therefore, by adding an anionic polymer, the negative charges of the anionic polymer cancel the electrostatic repulsive force acting between the colloidal particles, thereby enabling effective flocculation and sedimentation.

When the hydrogen sulfide removal agent is produced on an industrial scale, it is preferable to perform gravitational sedimentation using a flocculation-sedimentation apparatus (thickener).

### (3) Solid-Liquid Separation Process

Next, the iron hydroxide-containing slurry obtained in the flocculation and sedimentation process is subjected to solid-liquid separation (solid-liquid separation process). As the slurry is dehydrated in this solid-liquid separation process, the iron hydroxide contained in the slurry gradually begins to change into iron oxyhydroxide. Therefore, the solid content obtained by the solid-liquid separation (also referred to as a dewatered cake) contains iron oxyhydroxide. Accordingly, in the present specification, the solid content is referred to as an iron oxyhydroxide-containing cake.

The method of performing the solid-liquid separation is not particularly limited, but when the hydrogen sulfide removal agent is produced on an industrial scale, it is preferable to use a filter press.

### (4) Drying Process

Subsequently, the iron oxyhydroxide-containing cake is dried to obtain iron oxyhydroxide (drying process). As described above, Fe exists in the aqueous solution as iron hydroxide at the time of neutralization. However, as water is removed by the solid-liquid separation process and the drying process, dehydration condensation occurs, thereby converting the iron hydroxide into amorphous iron oxyhydroxide (FeOOH).

The composition of the finally obtained hydrogen sulfide removal agent greatly varies depending on the drying conditions in the drying process. For example, in order to convert iron hydroxide into amorphous iron oxyhydroxide, it is necessary to sufficiently remove water. In order to sufficiently remove water, it is necessary to increase the drying temperature and prolong the drying time.

On the other hand, amorphous iron oxyhydroxide crystallizes when excessively heated. Further, when excessively heated, it changes into iron oxide (Fe₂O₃), which is a crystalline component, by the reaction represented by the following equation (4). Therefore, in order to retain amorphous iron oxyhydroxide, it is necessary to lower the drying temperature and shorten the drying time.

2FeOOH → Fe₂O₃ + H₂O (4)

Accordingly, the drying conditions may be adjusted such that a hydrogen sulfide removal agent having a desired composition is obtained. Specific drying conditions vary depending on the production scale and the like. For example, when drying about 100 g of the iron oxyhydroxide-containing cake, the following conditions are preferable.
- When the drying temperature is 100 °C or more and 150 °C or less, the drying time is preferably 3 to 10 hours.

- When the drying temperature is 60 °C or more and less than 100 °C, the drying time is preferably 10 to 24 hours.
- When the drying temperature is 30 °C or more and less than 60 °C, the drying time is preferably 24 to 72 hours.
- When heating is not performed, vacuum drying is preferable.

With the above method, an iron oxyhydroxide-based hydrogen sulfide removal agent can be obtained. The obtained iron oxyhydroxide-based hydrogen sulfide removal agent is usually in powder form and can be used as a hydrogen sulfide removal agent as it is.

After the drying process, treatments such as grinding and molding may optionally be performed. When molding is performed, a molding additive (binder) may optionally be added. By using a molded product formed using the molding additive as the hydrogen sulfide removal agent, gas can easily flow through the product compared with a case where the hydrogen sulfide removal agent is used in powder form as it is. In addition, since the iron oxyhydroxide-based hydrogen sulfide removal agent of the present disclosure contains a crystalline component, a molded product having sufficient strength can be obtained with a relatively small amount of the molding additive added. Any molding additive may be used, and for example, either or both of bentonite and montmorillonite may be used.

### EXAMPLES

The present disclosure will be described in detail below based on examples, although the present disclosure is not limited to these examples.

### (Example No. 1)

A hydrogen sulfide removal agent was produced using an aqueous solution containing Fe₂O₃ and FeCl₂. Specifically, while bubbling air through the aqueous solution, CaCO₃ and Ca(OH)₂ were added to the aqueous solution to perform neutralization. By performing the bubbling, divalent iron ions present in the aqueous solution can be oxidized by air into trivalent iron ions. Next, an anionic polymer flocculant was added to flocculate and sediment the precipitate. After removing the supernatant, solid-liquid separation was performed by filtration. Subsequently, drying was performed under conditions of a drying temperature of 60 °C and a drying time of 24 hours.

### (Example No. 2)

A hydrogen sulfide removal agent was produced using an aqueous solution containing FeCl₃. Specifically, first, Ca(OH)₂ was added to the aqueous solution to perform neutralization. Next, an anionic polymer flocculant was added to flocculate and sediment the precipitate. After removing the supernatant, solid-liquid separation was performed by filtration. Subsequently, drying was performed under conditions of a drying temperature of 60 °C and a drying time of 24 hours.

### (Comparative Example No. 3)

For comparison, a conventional hydrogen sulfide removal agent composed of crystalline iron oxyhydroxide was used as Comparative Example No. 3. Specifically, a commercially available crystalline α-FeOOH (goethite) reagent was used.

### (Comparative Example No. 4)

For comparison, a conventional hydrogen sulfide removal agent composed of amorphous iron oxyhydroxide was used as Comparative Example No. 4. Specifically, sodium hydroxide was dropwise added to an aqueous Fe(ClO₄)₃ solution to perform neutralization, and the precipitate was filtered. Next, the obtained filtrate residue was vacuum-dried at room temperature to obtain a hydrogen sulfide removal agent composed of amorphous iron oxyhydroxide.

### (Comparative Example No. 5)

For comparison, one commercially available hydrogen sulfide removal agent was evaluated in the same manner as in the other Examples.

### (Comparative Example No. 6)

For comparison, one commercially available hydrogen sulfide removal agent (a product different from the hydrogen sulfide removal agent used in No. 5) was evaluated in the same manner as in the other Examples.

### (Comparative Example No. 7)

Waste liquid discharged from steelworks was subjected to solid-liquid separation by filtration. Subsequently, drying was performed under conditions of a drying temperature of 60 °C and a drying time of 24 hours.

### (Comparative Example No. 8)

Waste liquid discharged from steelworks was subjected to solid-liquid separation by filtration. Subsequently, drying was performed under conditions of a drying temperature of 60 °C and a drying time of 24 hours.

For each of the hydrogen sulfide removal agents obtained as described above, the content of the crystalline component, the content of amorphous iron oxyhydroxide, and the specific surface area were measured. The measurement results are listed in Table 1. Specific measurement methods were as follows.

### (Content of Crystalline Component)

The content of the crystalline component contained in the obtained hydrogen sulfide removal agent was measured by X-ray diffraction measurement. Specifically, the content was calculated from the detected intensity of diffraction lines. In addition, the individual contents of crystalline FeOOH and crystalline Fe₂O₃ were also determined. Table 1 lists the content of crystalline FeOOH, the content of crystalline Fe₂O₃, the content of other crystalline components, and the total amount of crystalline components. For some of the "other crystalline components," the specific components and their contents are also listed in Table 1.

### (Content of Amorphous Iron Oxyhydroxide)

The content of iron oxyhydroxide was determined using the Karl Fischer titration method according to the following procedure.

First, combined water (CW) was quantified with the Karl Fischer titration method. Specifically, the sample (hydrogen sulfide removal agent) was dried at 105 °C to remove adsorbed moisture. Subsequently, the amount of combined water released by heating at 950 °C was measured with the Karl Fischer titration method. The amount of combined water measured in this manner is the amount of H₂O desorbed from iron oxyhydroxide by the reaction represented by the following equation (6):

2FeOOH → Fe₂O₃ + H₂O (6)

From the measured amount of combined water, the content of iron oxyhydroxide was calculated based on the stoichiometric ratio in the equation (6) above. The content of amorphous iron oxyhydroxide was determined by subtracting the content of crystalline iron oxyhydroxide determined by the above-described X-ray diffraction measurement from the obtained content of iron oxyhydroxide.

### (Specific Surface Area)

The specific surface area of the hydrogen sulfide removal agent was measured by nitrogen adsorption-desorption measurement. Specifically, first, vacuum degassing was performed at room temperature for 12 hours before measurement. Subsequently, adsorption-desorption measurement was performed using nitrogen gas to obtain an adsorption isotherm. The temperature during the measurement was set to -196 °C. Next, the surface area per unit weight (specific surface area) was calculated by Brunauer-Emmett-Teller (BET) analysis of the obtained adsorption isotherm.

Next, the sulfur adsorption amount per unit weight and the sulfur adsorption amount per unit surface area of the obtained hydrogen sulfide removal agent were evaluated with the following method. The measurement results are listed in Table 1.

First, the obtained hydrogen sulfide removal agent was sieved to obtain a sample having a particle size of 0.5 mm to 1.0 mm. Next, 1 cm³ of the sample was packed into a reaction tube having an inner diameter of 4 mm, and the temperature of the reaction tube was controlled to 30 °C using a thermostatic chamber. H₂S diluted with N₂ to 7000 ppm was introduced from the inlet of the reaction tube, and gas discharged from the outlet side of the reaction tube was sampled every 15 minutes. The H₂S concentration in the gas was measured using a gas chromatograph.

For a while after the start of the test, all hydrogen sulfide was adsorbed in the reaction tube, and therefore no hydrogen sulfide was detected in the outlet-side gas. However, after a certain period of time, the hydrogen sulfide concentration contained in the outlet-side gas sharply increased. This is because the hydrogen sulfide removal agent in the reaction tube became unable to adsorb further hydrogen sulfide. Therefore, the sulfur adsorption amount per unit weight (adsorption capacity) was calculated from the amount of gas that had flowed until the hydrogen sulfide concentration contained in the outlet-side gas sharply increased.

Further, the sulfur adsorption amount per unit surface area was calculated by dividing the obtained sulfur adsorption amount per unit weight by the specific surface area of the hydrogen sulfide removal agent.

As can be seen from the results listed in Table 1, the hydrogen sulfide removal agents satisfying the conditions of the present disclosure contained a crystalline component and therefore had a smaller specific surface area than the hydrogen sulfide removal agent composed of amorphous iron oxyhydroxide (Comparative Example No. 4). Accordingly, the hydrogen sulfide removal agents of the present disclosure can be used stably because they have larger particle sizes and higher strength. In addition, despite having a smaller specific surface area, the hydrogen sulfide removal agents satisfying the conditions of the present disclosure exhibited excellent hydrogen sulfide removal performance similar to that of the hydrogen sulfide removal agent composed of amorphous iron oxyhydroxide (Comparative Example No. 4), with a sulfur adsorption amount per unit weight of about 10 wt%.

### [Table 1]

**Table 1**

| No. | Content (wt%) | | | | | | Properties | | | Remarks |
|---|---|---|---|---|---|---|---|---|---|---|
| | Amorphous FeOOH | Crystalline component | | | | | Specific surface area (m²/g) | Sulfur adsorption amount | | |
| | | (a) Crystalline FeOOH | (b) Crystalline Fe₂O₃ | (c) Others | | Total amount (=a+b+c) | | Per weight (wt%) | Per surface area (µmol/m²) | |
| 1 | 52 | 0 | 11 | 37 | CaCO₃ 6% | 48 | 131 | 10.1 | 24 | Example |
| 2 | 77 | 0 | 0 | 23 | Al₂O₃ 5% | 23 | 144 | 9.9 | 21.4 | Example |
| 3 | 0 | 100 | 0 | 0 | - | 100 | 16.7 | 0.3 | 5.73 | Comparative Example |
| 4 | 100 | 0 | 0 | 0 | - | 0 | 316 | 10.9 | 10.8 | Comparative Example |
| 5 | 42 | 0 | 0 | 58 | CaSO₄ 63% | 58 | 86.1 | 4.9 | 18 | Comparative Example |
| 6 | 35 | 2 | 0 | 63 | Fe₃O₄ 25% | 65 | 50.8 | 2.67 | 16.4 | Comparative Example |
| | | | | | SiO₂ 2% | | | | | |
| | | | | | CaSO₄ 12% | | | | | |
| 7 | 21 | 3 | 7 | 69 | SiO₂ 1% | 79 | 41.3 | 1.04 | 7.83 | Comparative Example |
| | | | | | CaCO₃ 3% | | | | | |
| 8 | 20 | 0 | 0 | 80 | Fe₃O₄ 25% | 80 | 17 | 0.72 | 13.2 | Comparative Example |
| | | | | | FeO 25 % | | | | | |
| | | | | | Fe 10% | | | | | |
| | | | | | SiO₂ 2% | | | | | |
| | | | | | CaCO₃ 5% | | | | | |

## Claims

1. An iron oxyhydroxide-based hydrogen sulfide removal agent comprising amorphous iron oxyhydroxide and a crystalline component, wherein
a content of the amorphous iron oxyhydroxide is equal to or greater than a content of the crystalline component.

2. The iron oxyhydroxide-based hydrogen sulfide removal agent according to claim 1, wherein the content of the amorphous iron oxyhydroxide is 50 wt% to 80 wt%.

3. The iron oxyhydroxide-based hydrogen sulfide removal agent according to claim 1 or 2, comprising crystalline iron oxide as at least a part of the crystalline component.

4. A method of producing an iron oxyhydroxide-based hydrogen sulfide removal agent, which is a method of producing the iron oxyhydroxide-based hydrogen sulfide removal agent according to any one of claims 1 to 3, comprising:
a neutralization process of neutralizing an aqueous solution containing iron ions to precipitate an iron component;
a flocculation and sedimentation process of flocculating and subjecting to sedimentation the iron component obtained in the neutralization process, and removing a supernatant to obtain an iron hydroxide-containing slurry;
a solid-liquid separation process of subjecting the iron hydroxide-containing slurry to solid-liquid separation to obtain an iron oxyhydroxide-containing cake; and
a drying process of drying the iron oxyhydroxide-containing cake to obtain iron oxyhydroxide.

5. The method of producing an iron oxyhydroxide-based hydrogen sulfide removal agent according to claim 4, wherein the aqueous solution containing iron ions is a waste liquid containing iron ions.

6. The method of producing an iron oxyhydroxide-based hydrogen sulfide removal agent according to claim 4 or 5, wherein a calcium compound is used as a neutralizing agent in the neutralization process.

7. The method of producing an iron oxyhydroxide-based hydrogen sulfide removal agent according to any one of claims 4 to 6, wherein the pH is adjusted to 6 to 8 in the neutralization process.

8. The method of producing an iron oxyhydroxide-based hydrogen sulfide removal agent according to any one of claims 4 to 7, wherein an anionic polymer is used as a flocculant in the flocculation and sedimentation process.

9. A desulfurization method of removing hydrogen sulfide contained in a gas using the iron oxyhydroxide-based hydrogen sulfide removal agent according to any one of claims 1 to 3.
